# EUROPEAN PATENT APPLICATION

(11) **EP 1 535 639 A1**
(43) Date of publication of application: **01.06.2005**
(21) Application number: 02754166.3
(22) Date of filing: 09.08.2002
(51) Int. Cl.: A61M 5/20

(54) **A SYRINGE**

(71) Applicant: Yang, Chang-Ming, Miaoli County, Taiwan (CN)
(72) Inventor: Yang, Chang-Ming, Miaoli County, Taiwan (CN)
(74) Representative: Viering, Jentschura & Partner
(86) International application number: PCT/CN2002/000553
(87) International publication number: WO 2004/014467

(57) **Abstract**

A syringe, which uses an air compressor to generate suction force to withdraw the air in the syringe and form vacuum suction to withdraw drugs or blood into the syringe to attain the objective of withdrawal. Besides, the compressed air by the air compressor can be charged into the syringe to generate push force to inject drugs or blood in the syringe into patient body or a blood storage test tube. Through pressing the blocking section of the needle socket, the elastic fixture in the blocking section is loosening off to allow the needle socket along with the needles retracting into the syringe body.

## Description

### BACKGROUND OF THE INVENTION

### 1. Field of the invention

The present invention is related to a syringe, which especially refers to the type that uses compressed air or vacuum suction for blood withdrawal or drug injection and has the needle retractable into the syringe after using.

### 2. DESCRIPTION OF THE PRIOR ART

Traditional needle injectors have several structural features. On top of syringe, there is a needle connector that connects to an injection needle. Inside syringe, there is a piston plunger, which is pulled outward to create negative pressure, so blood and drugs can be withdrawn into the syringe. Then, the piston plunger is pushed into the syringe to inject blood or drugs through nozzle. However, such traditional a syringes are operated manually through pulling or pushing the piston plunger, so vacuum suction or push force is generated for injection or withdrawal. As a result, manual pull or push may cause the withdrawal or injection process too fast or too slow due to uneven forces to pull or push the piston plunger, so the patients have numb feeling, discomfort or pain. Besides, due to inadvertent operation by medical staffs, patients have hematoma where the operation is applied. In addition, over-sampling or under-sampling may occur in blood withdrawal operation. Further, because the injection needles are installed on such traditional syringes, medical staffs need to be very careful when they are handling the used syringes to avoid needle sticks or bacteria infection.

Apparently, the above-mentioned traditional syringes still have many disadvantages and need further improvement.

In view of all kinds of disadvantages for the traditional a syringes, the inventor for the present invention was eager to make improvement and innovation. After many years of dedicated research and development, a syringe in the present invention is successfully developed.

### SUMMARY OF THE INVENTION

The objective for the present invention is to complete injection operation by using an air compressor to force a balloon inside the syringe body to push drugs or withdraw blood.

Another objective for the present invention is to use an air compressor to suck air from the balloon inside the syringe body and create vacuum suction for withdrawal operation.

Another objective for the present invention is to provide a mechanism that the needle can be retracted into the syringe by releasing the elastic element inside a blocking section that pushes the bottom of needle adapter.

A syringe to attain the above objectives will comprise:
a syringe body, at one end of which there is a needle connector, at inner edge of which there is a retainer, while at the other end of the syringe there is an opening;
a needle socket, which is installed inside the syringe and at the rear of which there is a blocking section, inside which there is a holding space for an elastic fixture;
a balloon, which is made of ductile material in long stripe and installed at outer edge of the rear of syringe;
a cylindrical plug, which is made of elastic material in cylindrical shape and inserted at the rear opening of syringe;
a nozzle, at the front of which there is a socket with several vent holes so the nozzle can pass through the cylindrical plug into a balloon, while at the rear of the nozzle there is a receptacle; and
a transport tubing, one end of which is installed into the receptacle of nozzle while the other end of which connects to an air compressor.

For the present invention, an air compressor is used to suck air from the balloon, so vacuum suction is generated inside the syringe to withdraw drugs or blood into the syringe; or an air compressor is used to charge air into the balloon, so the balloon injects the drugs or blood in the syringe into patient's body or blood storage tubes.

### BRIEF DESCRIPTION OF THE DRAWINGS

The drawings disclose an illustrative embodiment of the present invention that serves to exemplify the various advantages and objects hereof, and are as follows:
Fig. 1 is an illustration of a three-dimensional view of the syringe for the present invention.
Fig. 2 is a three-dimensional disassembling diagram for the syringe in the present invention.
Fig. 3 is a side view for the action of the syringe (1).
Fig. 4 is a side view for the action of the syringe (2).
Fig. 5 is a side view for the action of the syringe (3).
Fig. 6 is a side view for the action of the syringe (4).
Fig. 7 is a cross-sectional view for the needle socket.
Fig. 8 is a side view for the action of the needle socket.
Fig. 9 is a side view for the syringe in the first embodiment.
Fig. 10 is a side view for the elastic fixture.
Fig. 11 is a side view for the action of the elastic fixture.
Fig. 12 is a side view for the elastic fixture in the first embodiment.
Fig. 13 is a side view for the action of the elastic fixture in the first embodiment.
Fig. 14 is a side view for the elastic fixture in the second embodiment.
Fig. 15 is a side view for the action of the elastic fixture in the second embodiment.
Fig. 16 is a side view for the needle socket in the first embodiment.
Fig. 17 is a side view for the action of the needle socket in the first embodiment (1).
Fig. 18 is a side view for the action of the needle socket in the first embodiment (2).
Fig. 19 is a side view for the needle socket in the second embodiment.
Fig. 20 is a side view for the action of the needle socket in the second embodiment.
Fig. 21 is a side view for the needle socket in the third embodiment.
Fig. 22 is a side view for the action of the needle socket in the third embodiment (1).
Fig. 23 is a side view for the action of the needle socket in the third embodiment (2).
Fig. 24 is a side view for the needle socket in the fourth embodiment.
Fig. 25 is a side view for the needle socket in the fifth embodiment.
Fig. 26 is a side view for the needle socket in the sixth embodiment.

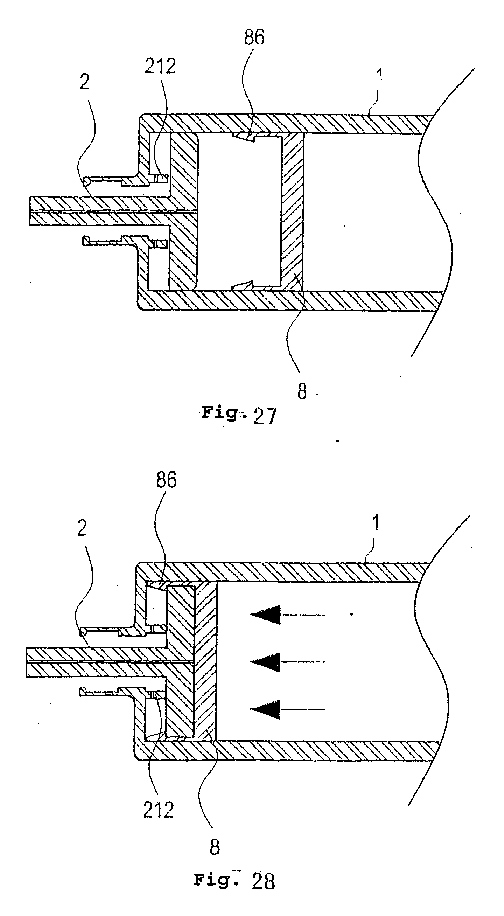

Fig. 27 is a side view for the needle socket in the seventh embodiment.
Fig. 28 is a side view for the action of the needle socket in the seventh embodiment (1).

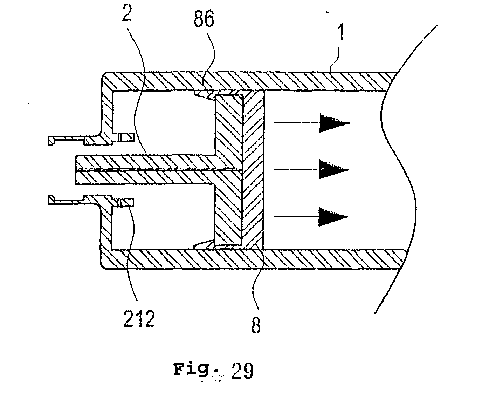

Fig. 29 is a side view for the action of the needle socket in the seventh embodiment (2).
Fig. 30 is a side view for the needle socket in the eighth embodiment.
Fig. 31 is the layout for the air compressor.
Fig. 32 is a side view for the nozzle in another embodiment.
Fig. 33 is a side view for the action of a syringe in the second embodiment (1);
Fig. 34 is a side view for the action of a syringe in the second embodiment (2);
Fig. 35 is a side view for a syringe in the third embodiment;
Fig. 36 is a three-dimensional disassembling diagram for the piston and the plunger in the third embodiment;
Fig. 37 is a three-dimensional view for a syringe in the fourth embodiment;
Fig. 38 is a three-dimensional view for a syringe in the fifth embodiment;
Fig. 39 is a three-dimensional view for a syringe in the sixth embodiment;
Fig. 40 is a three-dimensional view for the syringe body of a syringe in the first embodiment;
Fig. 41 is a three-dimensional view for the syringe body of a syringe in the second embodiment;
Fig. 42 is a three-dimensional view for the syringe body of a syringe in the third embodiment;
Fig. 43 is a side view for the needle socket in the ninth embodiment;
Fig. 44 is a side view for the needle socket in the tenth embodiment;
Fig. 45 is a side view for the action of a syringe in the seventh embodiment (1);
Fig. 46 is a side view for the action of a syringe in the seventh embodiment (2);
Fig. 47 is a side view for the action of a syringe in the seventh embodiment (3);

### DETAILED DESCRIPTION OF THE PREFERRED EMBODIMENT

For the present invention, a syringe uses air compressor to suck air from the syringe or charge air into the syringe, so push force or suction force is generated to enable the action of withdrawal or injection.

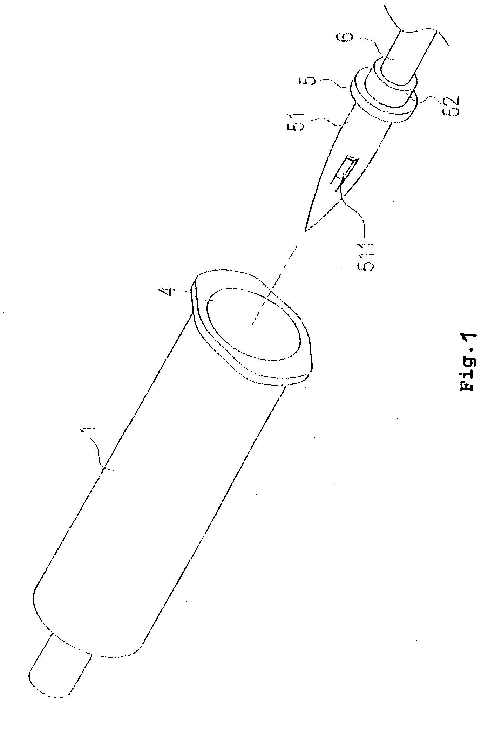

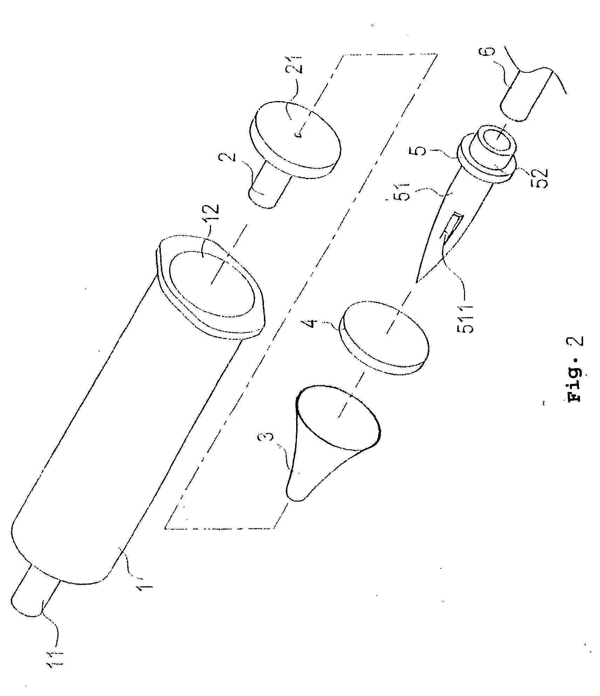

Please refer to Figs. 1 and 2. A syringe for the present invention mainly comprises:
a syringe body 1, at one end of which there is a needle connector 11, at the inner edge of which there is a retainer 111, while the other end of the syringe body 1 there is an opening 12;
a needle socket 2, which is installed in the syringe body 1 and the front of which is available for needle placement, while the rear of which there is a blocking section 21, inside which there is a holding space 22 for an elastic fixture 23, which is composed of two interlocking pushing stoppers 231, on the extension of one of which there is a bulging block 9, which extends along the outer edge of blocking section 21 and holds to the inner edge of the syringe body 1, with two pushing stoppers 231 stuck by adhesive 232;
a balloon 3, which is made of ductile material in a stripe;
a cylindrical plug 4, which is made of elastic materials in a cylindrical shape;
a nozzle 5, the front of which has a socket 51, which has several vent holes 511, and the rear of which has a receptacle 52; and
a transport tubing 6, one end of which is installed in the receptacle 52 of a nozzle 5 and the other end of which connects to air compressor;
the balloon 3 is installed at the outer edge of the syringe body 1 with a cylindrical plug 4 inserted into an opening 12 at the rear end of the syringe body 1, so with the exception of the part installed at outer edge of the syringe body 1, all the remaining part of the balloon 3 is pushed into the syringe body 1 by the cylindrical plug 4.

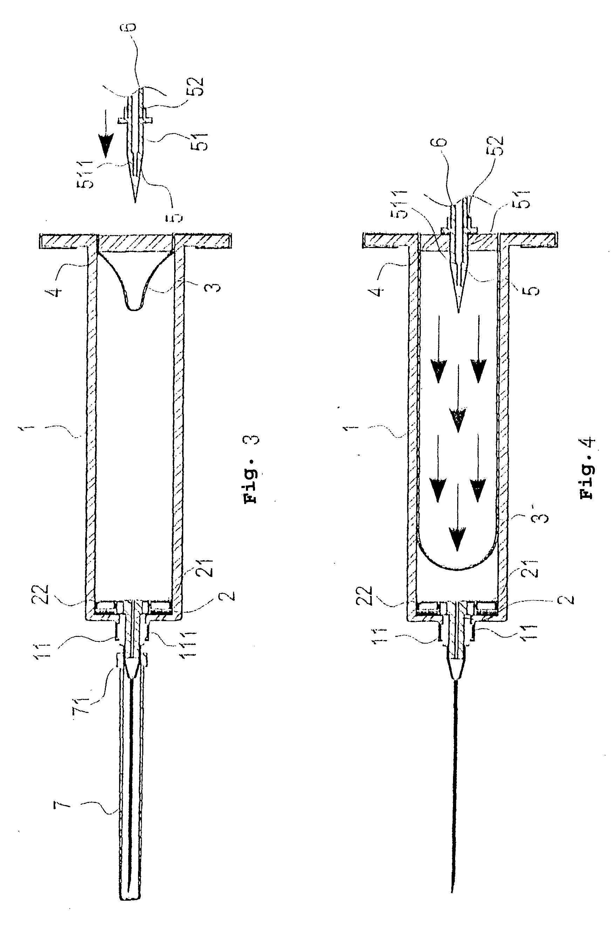

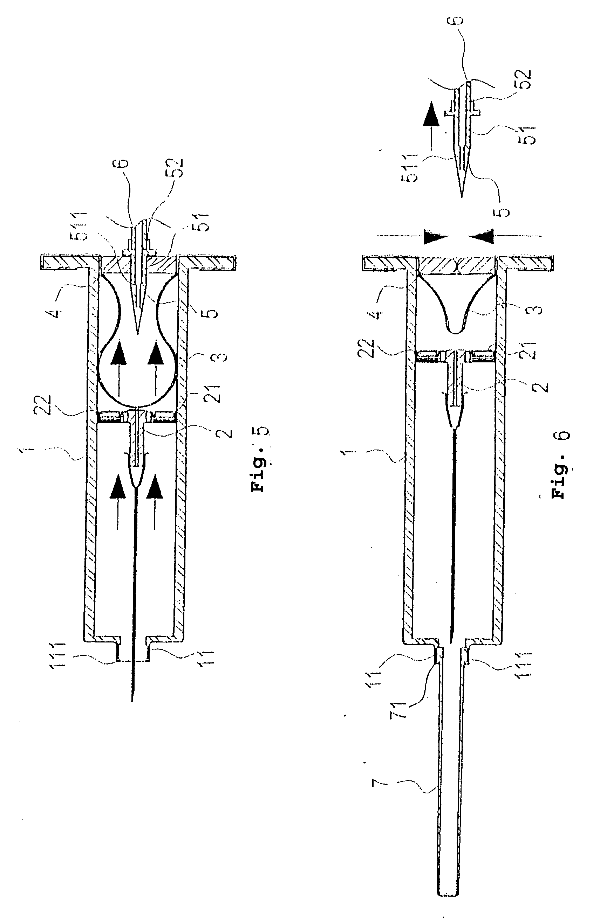

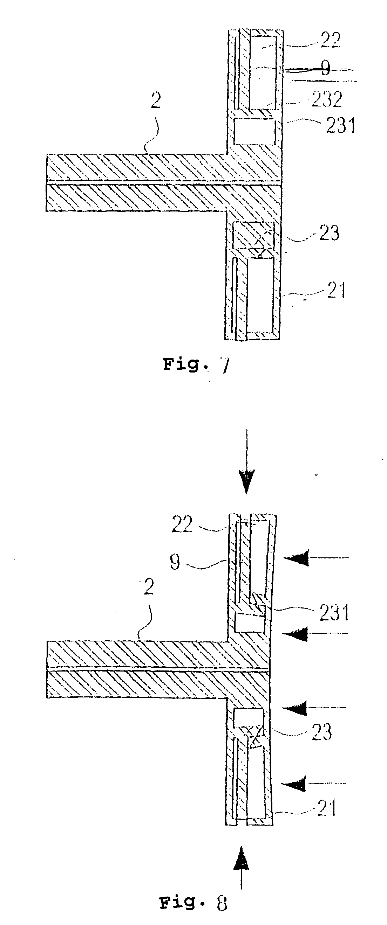

Please refer to Figs. 3, 4, 5, 6, 7, 8 and 9. When a syringe for the present invention is in use, first the injection needle and the needle cap 7 are installed on the needle socket 2, and then after removal of the needle cap 7 the nozzle 5 uses the front socket 51 for the cylindrical plug 4 to pass through and into the balloon 3. Afterward, the air compressor charges air through the transport tubing 6 and the vent holes 511 on the nozzle 5 into the balloon 3 of the syringe body 1. So the balloon 3 is inflated to tightly adhere to the inner wall of the syringe body 1. The needle in the needle socket 2 is inserted into a drug bottle or the blood vessel of a patient. Meanwhile, the air compressor is activated to suck out the air in the balloon 3 through vent holes 511 on the nozzle 5 and the transport tubing 6. Immediately, vacuum suction is generated in the syringe body 1 to suck the drugs or blood into the space formed by the syringe body 1 and the balloon 3. To this point, the action for withdrawing drugs or blood is completed. The following is to insert the needle into the patient body or the blood storage tube. With air compressor to charge air through the transport tubing 6 and the nozzle 5 into the balloon 3 of the syringe body 1. The balloon 3 is therefore inflated to push the drugs or blood existing in the space formed by the balloon 3 and the syringe body 1 into the patient body or blood storage tube through the needle in the needle socket. In the meantime, the air compressor continues to charge air into the balloon 3, which then continues inflation to pressure the blocking section 21 of the needle socket 2. When external forces press the two interlocking pushing stoppers 231 in the blocking section 21, they stick the adhesive and become mutually interlocked. The bulging block 9 in the pushing stopper 231 retracts into the holding space 22 in the blocking section 21 so the interfering force on the needle socket 2 and the inner wall of the syringe body disappears. The needle socket 2 and the needle retract into the syringe body and are locked in the retainer 111 of the needle connector 2 through a circular bulging block 71 at the outer edge of the needle cap 7. The needle cap 7 is fixed onto the syringe body 1. After the nozzle 5 is removed from the cylindrical plug 4, the hole left on the cylindrical plug 4 will be sealed up by itself due to material elasticity. Further, a circular elastic element 3 can be added between the syringe body 1 and the blocking section 21 of the needle socket 2. The elastic element 13 is used to increase the resistance of the blocking section 21. Optionally, the retainer 111 of the needle connector 2 is designed with screw threads so the needle cap 7 is screwed with the syringe body 1.

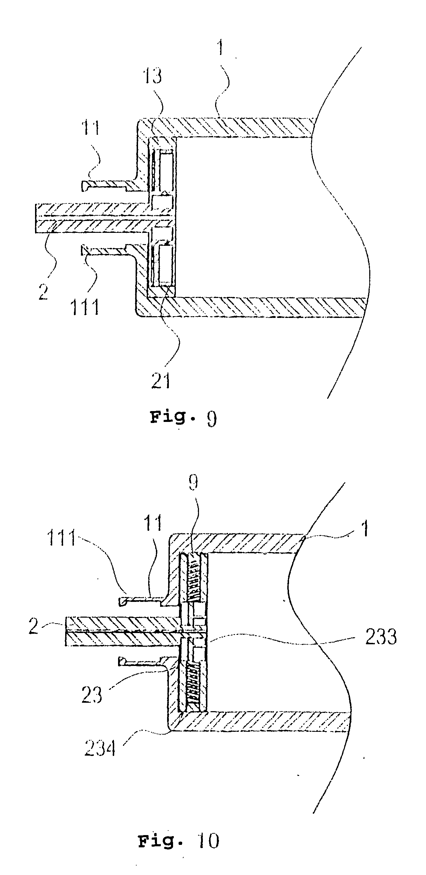

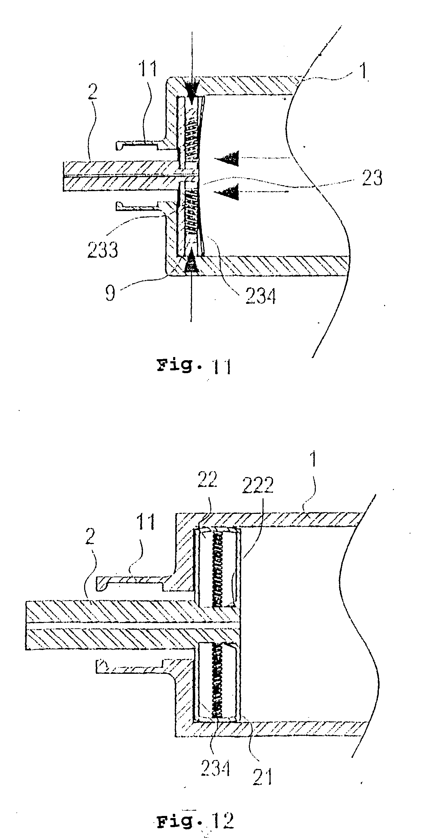

Please refer to Figs. 10 and 11. The elastic fixture 23 can also be composed of one or more than one T stoppers 233 and several springs 234. One end of the spring 234 has a bulging block 9, which extends along the outer edge of the blocking section 21 and holds onto the inner wall of the syringe body 1. The other end of the spring 234 holds onto the extended longer side of the T stopper 233. A groove 221 at the side of the holding space 22 is available for the placement of the extended longer side of the T stopper 233. When the balloon 3 pushes the elastic fixture 23, the extended longer side of the T stopper 233 is pushed into the groove 221. Therefore, the spring 234 along with the bulging block 9 retracts into the holding space 22. At the moment, the interfering force between the bulging block 9 and the inner wall of the syringe body disappears to allow the needle socket 2 retracting into the syringe body.

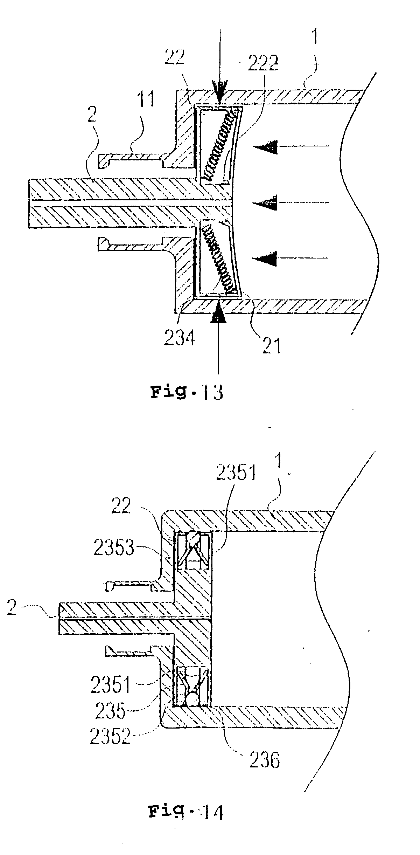

Please refer to Figs. 12 and 13. The elastic fixture 23 can be held onto the holding space 22 of the blocking section 21 by several springs 234. So the two sides of the blocking section 21 support the inner wall of the syringe body 1. Besides, there is a push block 222 for one side of each spring 234 in the holding space 22. When the balloon 3 pushes the elastic fixture 23, each push block 222 will separate each spring 234. As a result, the interfering force between the spring 234 and the syringe body disappears and the needle socket 2 is retracted into the syringe body.

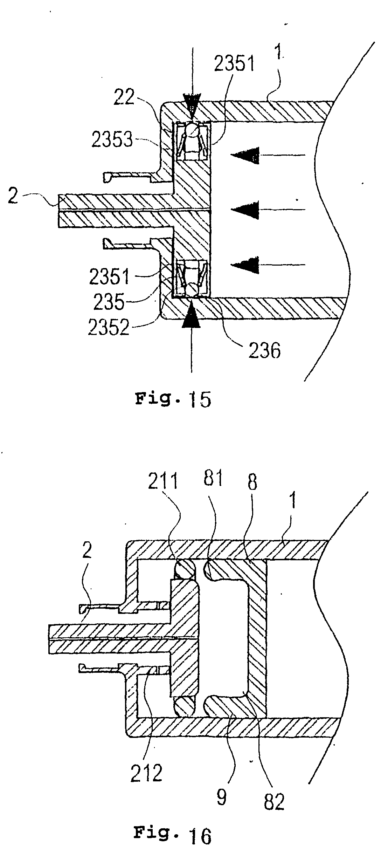

Please refer to Figs. 14 and 15. The elastic fixture 23 can also be composed of several clamp modules 235 and spring coils 236 made of materials like rubber. The clamp module 235 is composed of triangular elastic plate 2351 that is made of bent elastic material. The two ends of the opening 12 of the triangular elastic plate 2351 are bent into a circular clamping section 2352, inside which there is a lever 2353 that extends to the outer edge of the triangular elastic plate 2351. While the bottom of the triangular plate 2351 forms an elastic arch structure. In addition, the outer edge of the spring coil 236 holds onto the inner wall of the syringe body 1. While the inner edge of the spring coil holds onto the clamping section 2352 of the clamp module 235. When the balloon 3 pushes the elastic fixture 23, the lever 2353 is forced to open the two clamping sections 2352 of the triangular elastic plates 2351. Therefore, the elastic spring coils 2361 retract into the opening of the triangular elastic plate 2351. As a result, the interfering force on the spring coils 236 and the inner wall of the syringe body disappears.

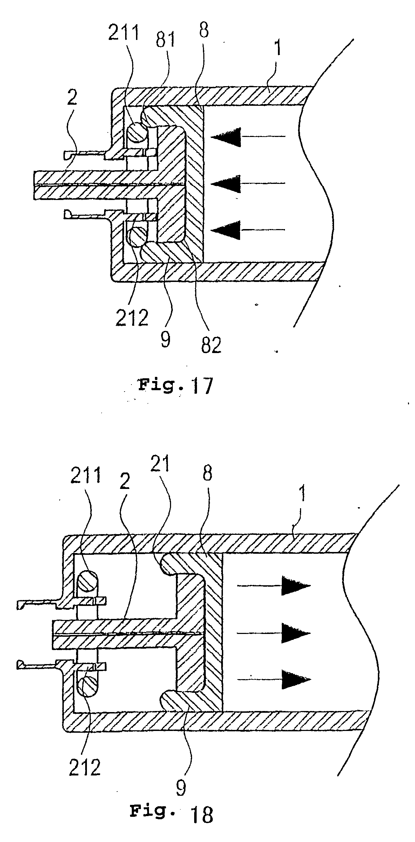

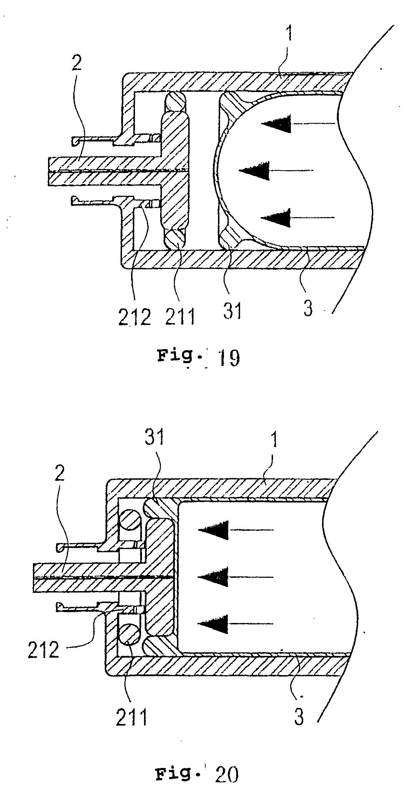

Please refer to Figs. 16, 17, 18, 19 and 20. The blocking section 21 of the needle socket 2 may not need the holding space 22 for the elastic fixture 23. At the outer edge of the blocking section 21 there are spring coils 211 made of elastic materials like rubber. Besides, one or more than one support 212 are installed between the syringe body 1 and the blocking section 21. A piston 8 is installed in the syringe body 1. A bulging block 9 is installed and surrounds the piston 8 at the position corresponding to the spring coil 211 and forms a clamping section 82 at the position between the piston and the bulging block 9. Besides, the inner edge of the bulging block 9 top has a spherical bulging block 81. When the piston 8 is pushed by air, the bulging block 9 of the piston 8 pushes the spring coils off the outer edge of the blocking section 21 so the blocking section 21 is placed in the clamping section 82. In the meantime, the spherical bulging part 81 surrounding the bulging block 9 is hooked into the front of the blocking section 21, so the blocking section 21 of the needle socket 2 is covered by the bulging block 9 in the clamping section 82. The piston 8 is pulled back by the negative pressure to allow the needle socket 2 and the syringe needle retracting into the syringe body 1. Further, the piston 8 can be replaced by the balloon 3, which front has a circular bulging part 31 to surround the balloon 3. When the balloon 3 is inflated and presses the blocking section 21 of the needle socket 2, the circular bulging part 31 pushes the spring coils 211 off the outer edge of the blocking section 21. Immediately, the resisting force formed through the spring coils 211 between the blocking section 21 and the syringe body 1 disappears to allow the needle socket 2 and the needle retracting into the syringe body. Alternatively, the spring coil can be made of non-elastic material.

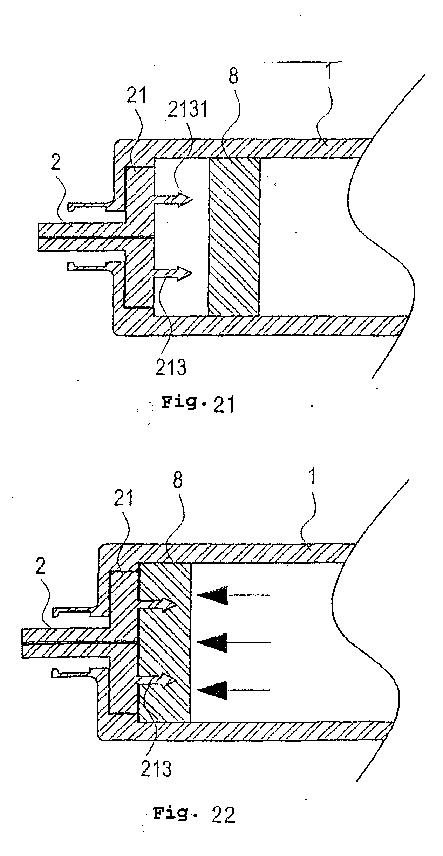

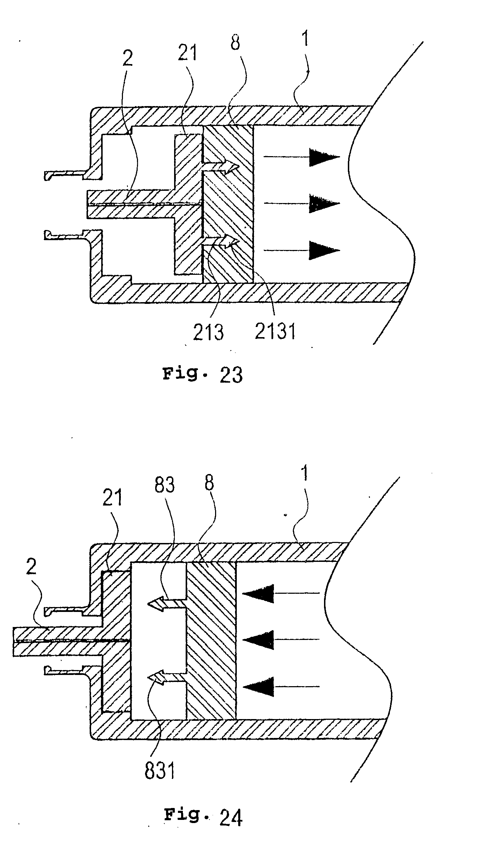

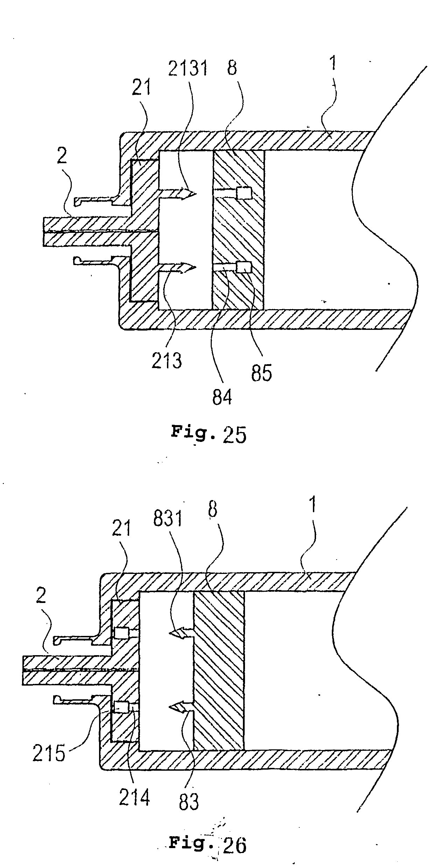

Please refer to Figs. 21, 22, 23, 24, 25 and 26. The rear of the blocking section 21 has several locking tenons 213 with reversed hooks 2131. There is a piston 9 of elastic material in the syringe body 1. When the piston 8 is pushed forward by air, the locking tenon 213 of the blocking section 21 will enter the piston 8 or pass through the piston 8. Meanwhile, the hole punched by the locking tenon 213 on the piston 8 is sealed by itself due to the elasticity and wraps around the locking tenon 213. The reversed hook 2131 of the locking tenon 213 locks the piston 8 and the blocking section 21. Then, the piston 8 is pulled out by negative pressure, so the needle socket 2 and needle are pulled into the syringe body 1. Besides, the blocking section 21 can also be made of elastic materials. The piston 8 has several locking tenons 83 with reversed hooks 831 across the blocking section 21. The piston 8 may be made of non-elastic materials too. The piston 8 has several guiding holes 84 of corresponding quantity and at corresponding position to the locking tenons 213. Besides, at the bottom of each guiding hole 84 there is a holding groove wider than the guiding hole 84. When the piston 8 is pressed by air, the locking tenon 213 on the blocking section 21 is forced to enter the holding groove 85 through the guiding hole 84 of the piston 8. The reversed hooks 2131 for each locking tenon 213 hold onto the wall of the holding groove 85, so the piston 8 combines with the blocking section 81. Then the piston 8 along with the needle socket 2 and the needle is pulled into the syringe body 1. Further, when the locking tenon 83 is installed on the piston 8, the blocking section 21 can be made of non-elastic materials. Besides, several guiding holes 214 are made for the blocking section 21 in corresponding quantity and at the corresponding position to the locking tenon 83 of the piston 8. At the bottom of each guiding hole 214 there is a holding groove 215 wider than the guiding hole 214. Further, a pathway can be made in the holding groove 85 or 215 for the above-mentioned piston 8 or blocking section 21 to connect to the needle socket 2 as passage of drugs.

Please refer to 27, 28 and 29. The blocking section 21 can also be made of elastic materials. The outer edge of the blocking section 21 connects to the inner wall of the syringe body 1. The balloon 3 is replaced by a piston 8. The outer edge of the piston 8 corresponding to the blocking section 21 has surrounding locking hooks 86. When the piston 8 is pushed by air, because the blocking section 21 is made of elastic materials, the locking hooks 86 of the piston 8 can push the blocking section 21 toward the syringe body 1 through the contact surface on the blocking section 21 and the inner wall of the syringe body 1. As a result, the locking hooks 86 pass through the outer edge of the blocking section 21 and lock onto the blocking section 21. Negative pressure is then used to pull the piston 8 backward. In the meantime, the locking hooks 86 are locked onto the blocking section 21. Hence, the piston 8 moves the needle socket 2 with the injection needle into the syringe body 1. Further, a pathway can be open in the holding groove on the above-mentioned piston 8 or blocking section 21 to connect to the needle socket 2 as drug passage.

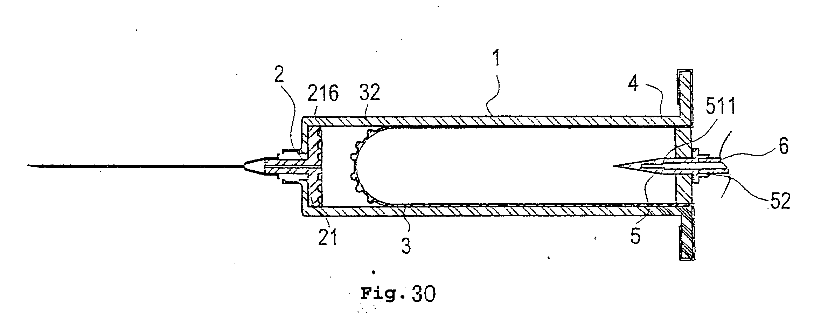

Please refer to Fig. 30. The blocking section 21 of the needle socket 2 may not need the holding space 22 for the elastic fixture 23. Instead, the rear of the blocking section 21 has several circular grooves 216. In addition, the outer edge of the balloon 3 has several spherical bulging parts 32 at the position corresponding to the circular grooves 216.

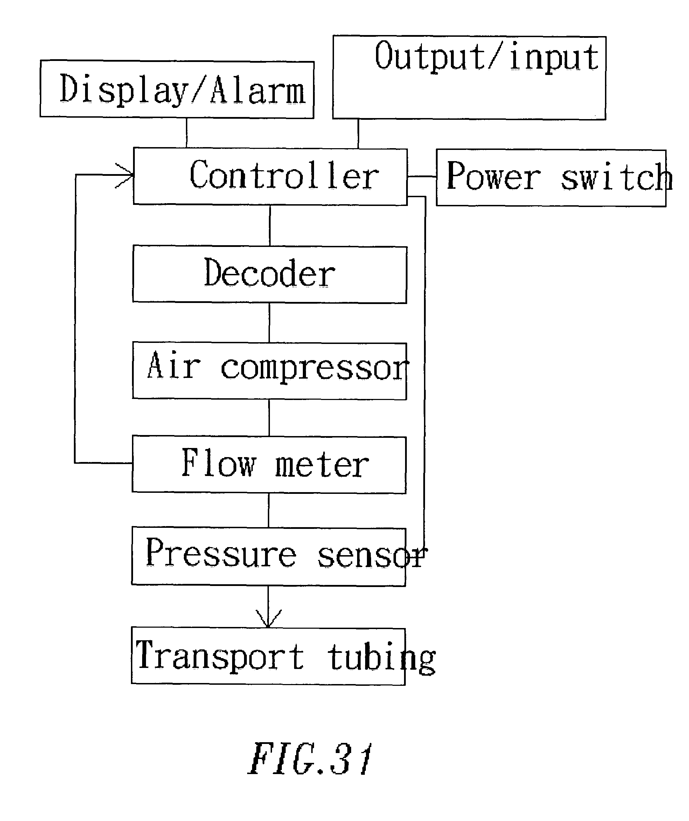

Please refer to Fig. 31. There are a display and an alarm on the air compressor. When injection and withdrawal are completed or clogged injection outlet or leakage is found, the alarm will sound to notify the medical staffs. Besides, the air compressor has a controller, a decoder and a flow meter to allow adjustable rate of injection or withdrawal or constant rate control. Furthermore, on the tope of the air compressor there is a pressure sensor, which allows detection of holes or leakage on the balloon 3 through the pressure variation in the balloon 3 when compressed air is charged into the balloon 3. Hence, when air compressor is operated to suck or deliver air, whether there is clogged injection outlet or leakage can be determined by the internal pressure variation in the balloon 3. When special conditions arise (such as patients in coma or balloon rupture...etc.), the air compressor power can be immediately switched off to interrupt the withdrawal or injection action. When blood withdrawal is performed on artery, first the balloon 3 is inflated to contact the inner wall of the syringe body 1, and then the injection needle along with the needle cap 7 is placed on the needle socket 2. Afterward, when the needle cap 7 is pressed onto the patient skin, the pressure sensor will read different variation on artery than normal blood vessels. At this moment, the needle cap 7 is removed for blood withdrawal on artery. The pressure sensor can still read the signal when the needle is inserted into the artery, so the medical staffs are guided to continue the blood withdrawal. Further, the air compressor has an output/input device that is connectable to computer, PDA or mobile phone. Furthermore, the air compressor can be replaced by hydraulic pump. Alternatively, the air compressor can have a transport tubing 6 to connect to an inflatable thin pad, which can be wrapped around the upper place of the injection position. When the thin pad is charged with air from the air compressor, it inflates to replace the traditional wrapping rubber band.

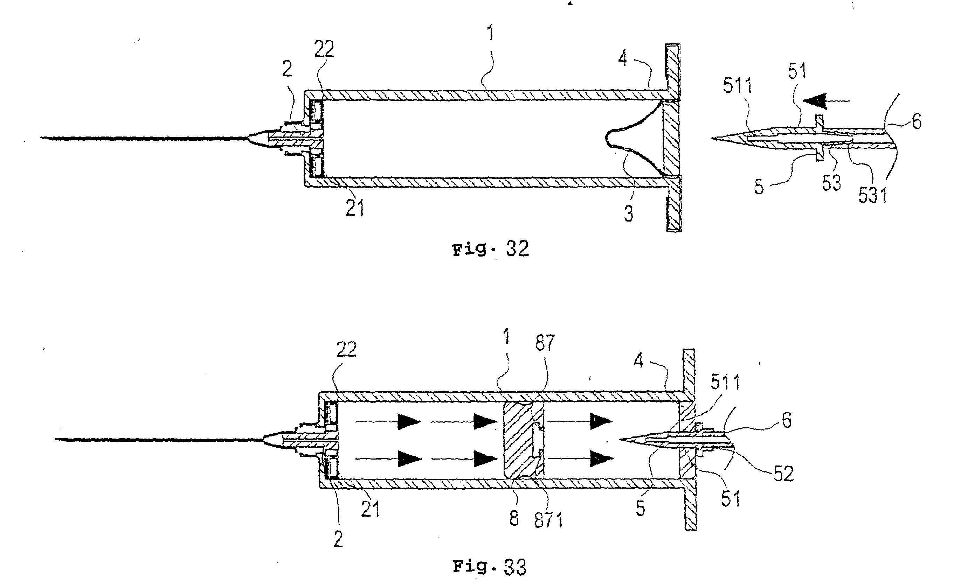

Please refer to Fig. 32. The rear receptacle 52 of the nozzle 5 can be replaced by a penetration section 53. The outer edge of the penetration section 53 extends backward to form a slope, on which there are several locking bulging blocks 531 to allow tight match between the bulging blocks 531 and the inner wall of the transport tubing 6 when the tubing 6 is inserted into the penetration section 53.

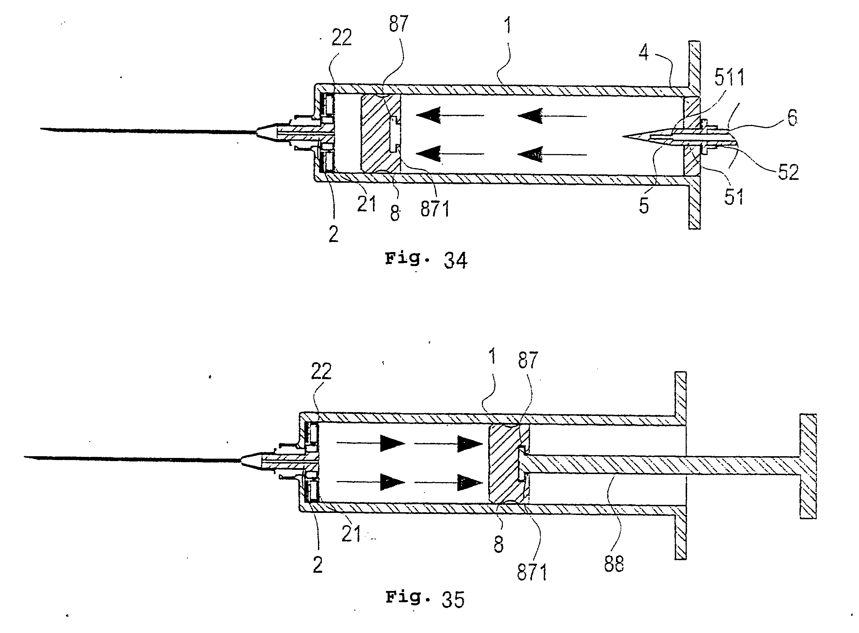

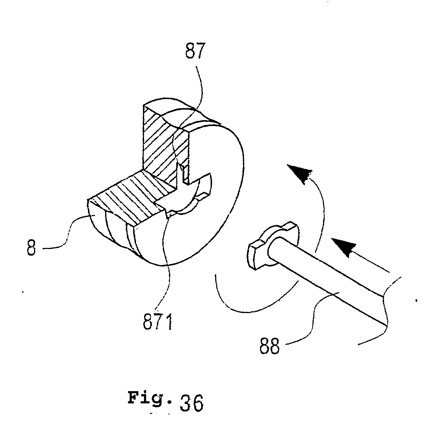

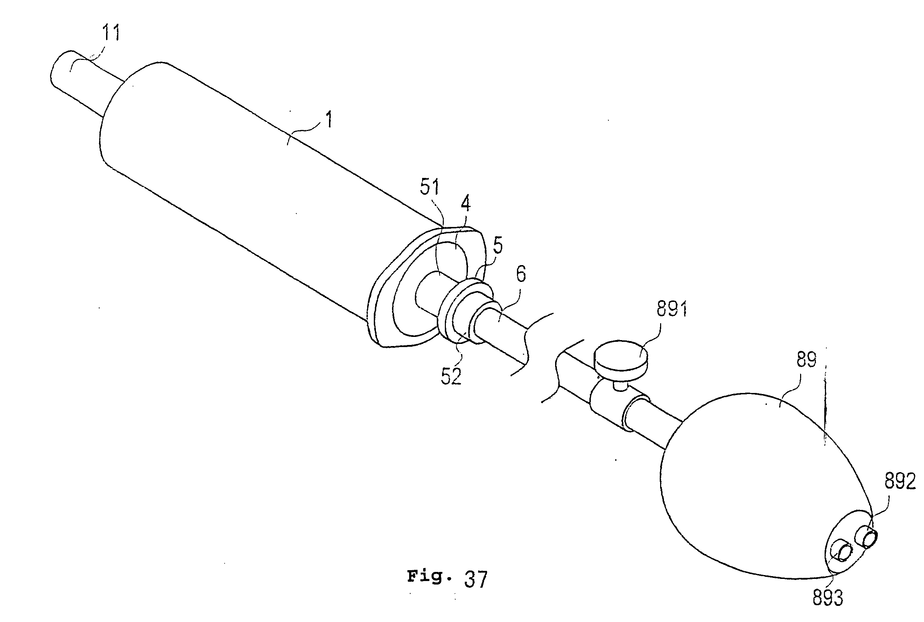

Please refer to Figs. 33, 34, 35, 36 and 37. A piston 8, which outer edge fits the inner wall of the syringe body 1 tightly, can replace the balloon 3 of a syringe in the present invention. So an airtight chamber is formed between the piston 8 and the cylindrical plug 4. At the rear of the piston there can be a holding groove 87, which has several bulging blocks 871 at the opening. The piston 8 is placed into the syringe body 1 through the opening at the rear. When a syringe for the present invention is in use, the needle is inserted into drug bottle. Air compressor will suck out the air in the closed airtight chamber. So the chamber is in a negative pressure. The plunger will pull the piston 8 backward to create vacuum suction force between the syringe body 8 and the needle socket 2. The drug in the drug bottle is withdrawn into the syringe body 1 through the needle and stays in the space between the needle socket 2 and the piston 8. Then, a syringe is inserted into patient body. Air is pumped into the syringe body 1 by air compressor to create a pushing force that the piston 8 moves the drug toward the needle head. The piston 8 helps injection of the drug in the syringe body 1 through the needle head to the patient body ; When power failure occurs or air compressor faults, the cylindrical plug 4, nozzle 5, and transport tubing 6 can also be pushed into the guiding holes 84 of piston 8 by piston tappet 88 and be fixed , or to substitute it by the manual compressor 89, There is an exhaust valve 891 at the connection of the manual compressor 89 and the transport conduit 6. At the rear of the manual compressor there are a unidirectional inlet valve 892 and a unidirectional exhaust valve 893.

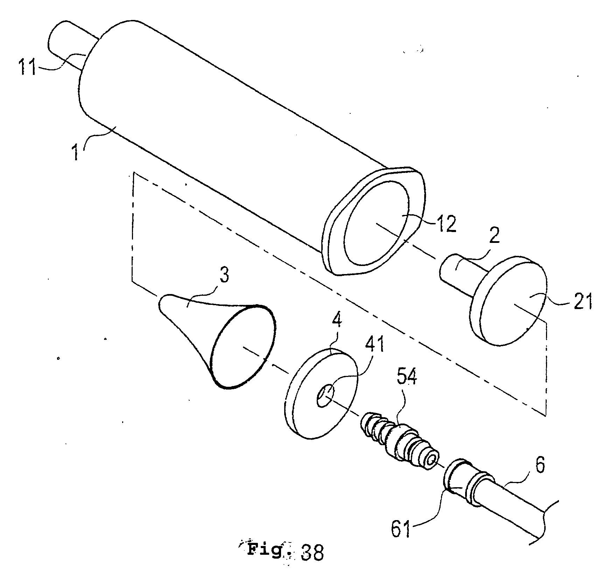

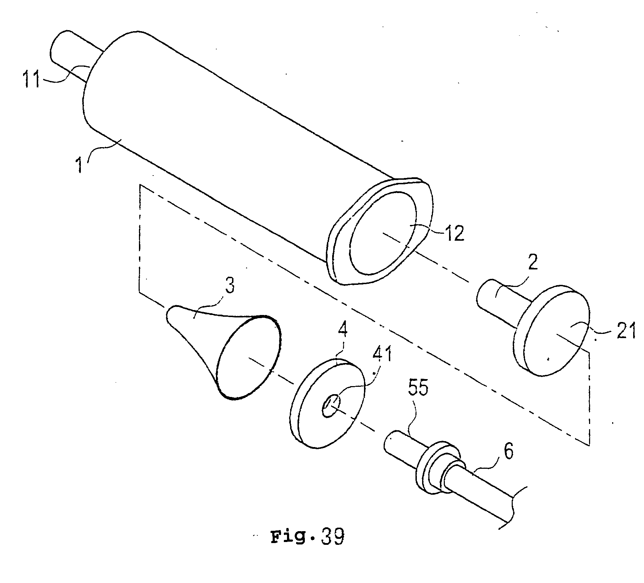

Please refer to Figs. 38 and 39. The cylindrical plug 4 for the present invention can also be made of non-elastic material. Besides, on the cylindrical plug 4 there are vent holes 41 and a quick connector 54 is used to replace the nozzle 5. On the transport tubing 6, there is a connector base 61 to connect to the quick connector 54. Further, a normal connector 55 can replace the said quick connector 54. The transport tubing 6 then connects to the rear of the normal connector 55.

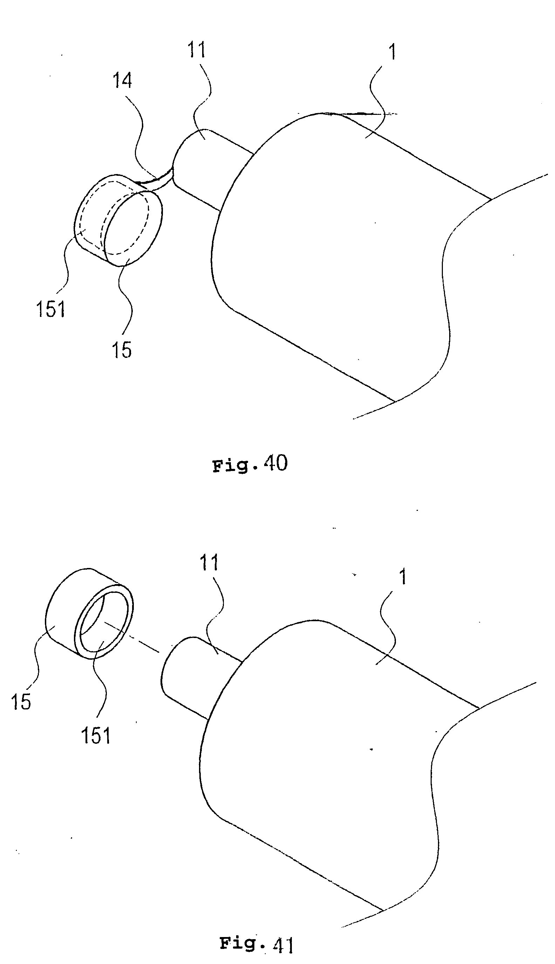

Please refer to Figs. 40, 41 and 42. At the front outer edge of the syringe body 1 there cab be an elastic connection end 14, the other end of which connects to a cap, inside which there is a receptacle 151 installed at the front outer edge of the cap 15. Further, the cap 15 does not need to connect to the syringe body 1. The receptacle 151 in the cap 15 can be replaced by a bulging holder 152, which can be placed in the needle connector 2 at the front of the syringe body 1.

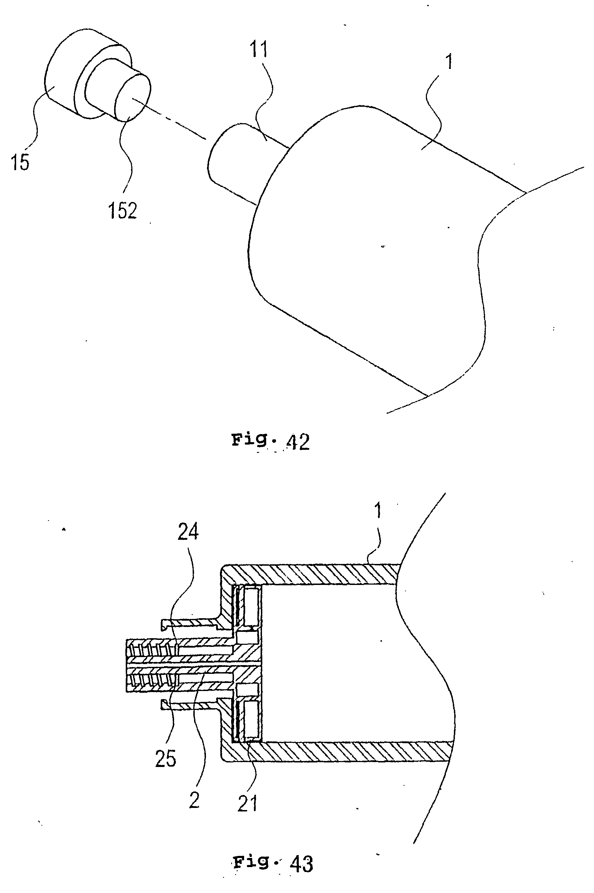

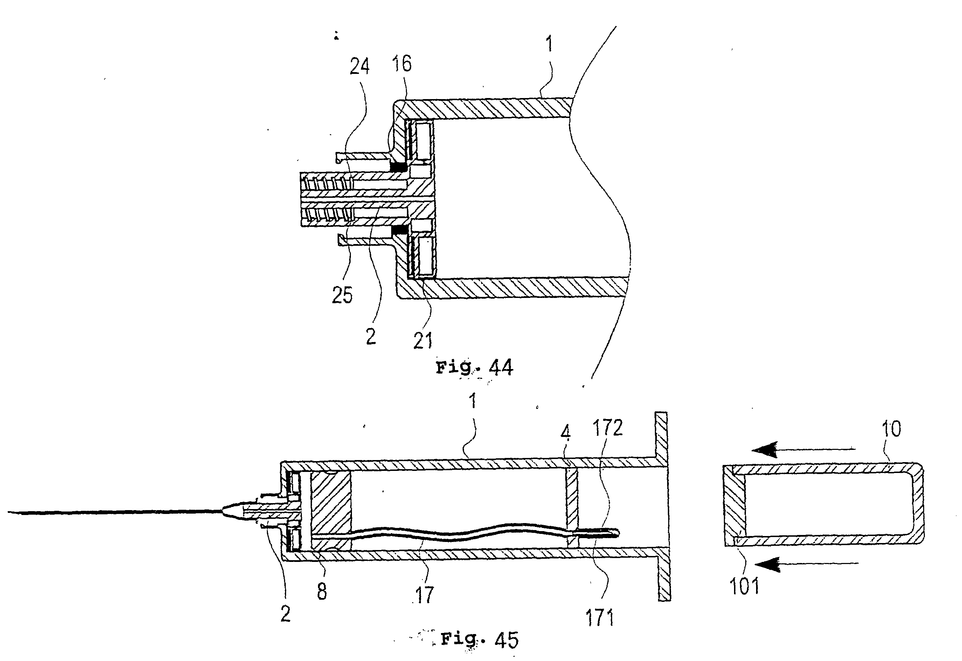

Please refer to Figs. 43 and 44. At the outer edge of the needle socket 2 there is an exterior ring 24, which has screw threads 25 for tightening the needle. In addition, the rear of the retainer 111 has an elastic element 16, which holds onto the outer edge of the exterior ring 24. When the needle socket 2 retracts into the syringe body 1, the elastic element 16 bounces to the original position of the exterior ring 24, so the needle socket 2 cannot return to the original position due to blockage by the elastic element 16. When the elastic element 16 bounces back to the original position of the exterior ring 24, the front of the needle cap 7 can be placed in the middle of the elastic element 16 to seal the injection needle into the syringe body 1.

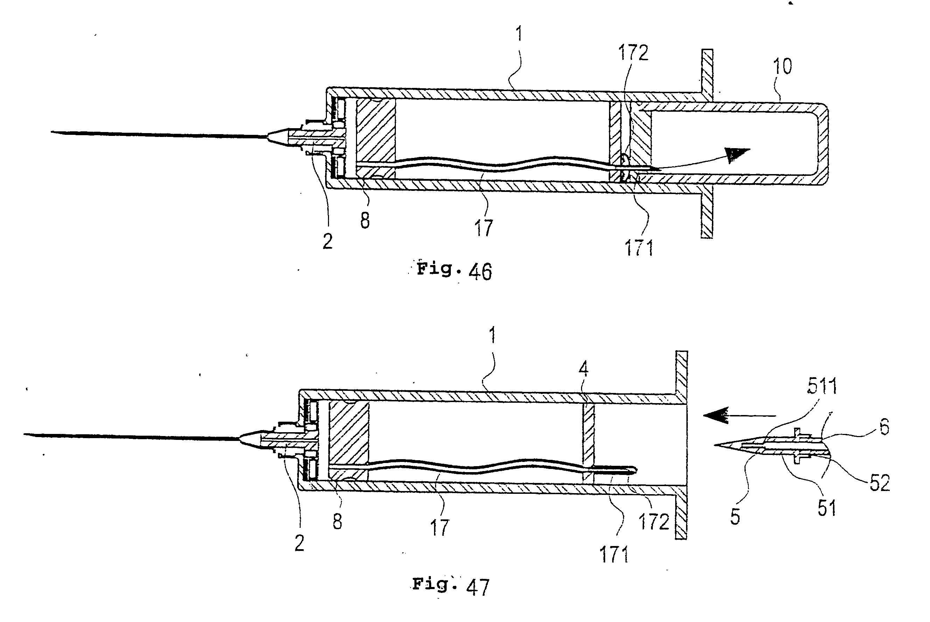

Please refer to Figs. 45, 46 and 47. The balloon 3 for the present invention can be replaced by a piston 8, between which and the cylindrical plug 4 there is a soft tubing 17, one end of which connects to the piston 3 and passes through the piston 3, while the other end of which passes through the cylindrical plug 3 and connects to a needle head 171, which outer edge has an exterior cover 172 made of compressible materials. When blood sampling is conducted, it only needs to push a test tube 10 into the syringe body 1 and allow the needle head 171 to penetrate the soft cork 101 of the test tube 10 and punch the exterior cover 172. In the meantime, due to negative pressure in the test tube 10, the blood is withdrawn through the soft tubing 17 directly from the needle to the test tube 10. When repeated blood sampling is required, there is no need to remove the needle from the patient body, but to replace the test tube 10. When the blood sampling is completed, the nozzle 5 is inserted into the cylindrical plug 4 and air is charged into the syringe body 1 through the nozzle 5. Hence, the air pushes the piston 17 to break up the connection between the needle socket 2 and the syringe body 1. Finally, the needle socket 2 along with the injection needle retracts into the syringe body 1.

By comparing with the above-mentioned methods or other traditional methods, a syringe for the present invention has the following advantages:
1. For a syringe for the present invention, an air compressor is used to make a balloon to push drugs or blood in the syringe body to a test tube, patient body or a test tube for blood storage through an injection needle.
2. For a syringe for the present invention, an air compressor is used to suck air out of the balloon from a nozzle through a transport tubing and form vacuum suction inside the syringe body, so blood or drugs can be withdrawn from the patient body or a drug bottle into the syringe body through the needle socket.
3. For a syringe for the present invention, a display or an alarm connecting to an air compressor will sound to notify medical staffs when injection or withdrawal is completed or clogged injection outlet or leakage is found.
4. For a syringe for the present invention, a controller, a decoder and a flow meter connecting to an air compressor are used to adjust the injection or withdrawal rate and to control timing and constant quantity.
5. For a syringe for the present invention, a pressure sensor connecting to an air compressor is used to determine whether a hole or leakage occurs in the balloon through the detection of pressure variation in the balloon when air is compressed into the balloon by the air compressor.
   Similarly, clogged injection outlet or leakage can be determined by pressure variation in the balloon when the air compressor is sucking or charging air.
6. For a syringe for the present invention, the power switch for the air compressor can be used to stop the action of withdrawal or injection.
7. For a syringe for the present invention, an output/input device on the air compressor is available for connection with computer, PDA or mobile phone.
8. For a syringe for the present invention, the elastic fixture in the blocking section can be loosen by pressing the blocking section of the needle socket, so the needle socket along with the injection needle can retract into the syringe body and medical staffs or patients can be protected from infection due to needle stick.
9. For a syringe for the present invention, a locking tenon in the needle cap is locked with the retainer of the needle connector on the syringe body to prevent dangers due to injection needle sticking from the syringe body.
10. For a syringe for the present invention, destroying the connection between the needle socket and the syringe body cannot reuse the needle.
11. For a syringe for the present invention, the artery injection position can be located because the pressure sensor on the air compressor can read the signal variation when the syringe is pressed onto the patient skin, so medical staffs can be guided for blood withdrawal on artery.
12. For a syringe for the present invention, the balloon is replaced by a piston. A soft tubing is installed between the piston and the cylindrical plug. One end of the soft tubing connects to a needle head for repeatable blood sampling.
Many changes and modifications in the above-described embodiment of the invention can, of course, be carried out without departing from the scope thereof. Accordingly, to promote the progress in science and the useful arts, the invention is disclosed and is intended to be limited only by the scope of the appended claims.

## Claims

1. A syringe, using an air compressor to suck air out of said a syringe and creating vacuum suction for withdrawal of drugs or blood, or using an air compressor to charge air into said a syringe so push force generated to inject drugs into patient's body.

2. A syringe of Claim 1, wherein said a syringe comprises a syringe body, a needle socket, a balloon, a cylindrical plug, a nozzle and a transport tubing, with a needle connector at one end and an opening at the other end.

3. A syringe of Claim 1, wherein an air compressor of said a syringe connects to a display and an alarm, said alarm would sound to notify medical staffs when injection and withdrawal are completed or clogged injection outlet or leakage is found.

4. A syringe of Claim 1, wherein said air compressor of said a syringe connects to a controller, a decoder and a flow meter, so an injection or withdrawal rate can be adjusted and time control and constant rate can be operated.

5. A syringe of Claim 1, wherein said air compressor of said a syringe can be replaced by a manual air compression structure, said manual air compression structure has an exhaust valve at a connection with a transport tubing and has an unidirectional inlet valve and an unidirectional exhaust valve at a rear.

6. A syringe of Claim 1, wherein said air compressor of said a syringe has a pressure sensor, said pressure sensor can detect pressure variation in said balloon and whether rupture or leakage exists in said balloon when air is charged into said balloon, so whether said injection outlet is clogged or has leakage can be determined when said air compressor is sucking or compressing air, and similarly said pressure sensor can read artery pulses to guide medical staffs to perform blood sampling when said a syringe is pressed onto patient's skin.

7. A syringe of Claim 1, wherein said air compressor of said a syringe has a power switch, said power switch is switched off to interrupt withdrawal or injection action.

8. A syringe of Claim 1, wherein said air compressor of said a syringe has an output/input device connectable to computer, PDA or mobile phones.

9. A syringe of Claim 1, wherein a hydraulic pump can replace said air compressor of said a syringe.

10. A syringe of Claim 1, wherein said air compressor of said a syringe has an inflation thin pad connecting to said transport tubing to replace a rubber band tied on an upper place of an injection position.

11. A syringe of Claim 2, wherein an inner edge of said needle connector of said a syringe has a concave retainer.

12. A syringe of Claim 2, wherein said balloon of said a syringe is in a stripe of elastic materials and installed at an outer edge of the rear of said syringe body with non-external part placed in said syringe body.

13. A syringe of Claim 2, wherein said cylindrical plug of said a syringe is a cylinder of elastic materials and placed at an opening of the rear of said syringe body.

14. A syringe of Claim 2, wherein a front of said nozzle of said syringe has a socket for said plug to pass through said nozzle into said balloon and several vent holes are on said socket, the rear of said nozzle has a receptacle, said receptacle has a transport tubing, besides, the other end of said transport tubing connecting to said nozzle is installed on said air compressor.

15. A syringe of Claim 2, wherein said balloon of said syringe can be replaced by a piston, an outer edge of said piston fits tightly with an inner wall of said syringe body, so an air-tight closed chamber is formed between said piston and said cylindrical plug.

16. A syringe of Claim 2, wherein a front outer edge of said syringe can have an elastic connection end, the other end of said elastic connection end connects to a cap, said cap has a concave holding space that can fit the needle connector at a front of said syringe body.

17. A syringe of Claim 2, wherein said balloon can be replaced by a piston, between said piston and said cylindrical plug there is a soft tubing, one end of said soft tubing connects to said piston and passes through said piston, while the other end of said piston passes through said cylindrical plug to connect a needle head, at an outer edge of said needle head a cover of compressible material is installed.

18. A syringe of Claim 2, wherein the soft cork is for placement of the needle cap, the needle head cap can connect to a needle cap through inflation, the needle cap has a needle hole and a retainer, the retainer has a round hole; further, at the fringe of the syringe body there are several inflation conduits with one side connecting to the inflation jacket and the other side to the air compressor.

19. A syringe of Claim 11, wherein the rear of a retainer of said syringe body has a wrapped elastic element to bounce to a needle socket position when said needle socket retracts into said syringe body.

20. A syringe of Claim 12, wherein a circular elastic device is installed between said syringe body and a blocking section of said needle socket to increase resistance for said blocking section.

21. A syringe of Claim 12, wherein said blocking section does not need a holding space for said elastic fixture, said blocking section can be made of elastic materials to have its outer edge contact with an inner wall of said syringe body, while said balloon is replaced by a piston, at an outer edge of said piston corresponding to said blocking section there are locking hooks.

22. A syringe of Claim 12,inside the retainer of a syringe the holding space of the elastic fixation can be omitted. Instead, an air bag made of balloon material is used at the rear of the retainer. The outer edge of the air bag extends to cover the outer side of the retainer. At the rear of the air bag, there is one or more than one air conduits made of elastic material. Besides, there are several bulging parts on the inner wall of the syringe body.

23. A syringe of Claim 12, wherein said blocking section of said syringe may not need a holding space for said elastic fixture, however, at an outer edge of said blocking section there is a spring coil, in addition, between said syringe body and said blocking section there is one or more than one support, inside said syringe body, there is a piston, at one side of said piston corresponding to said spring coil a bulging block exists at an outer edge of said piston to form a clamping section, a top inner edge of said bulging block has a spherical bulging part.

24. A syringe of Claim 12, wherein said blocking section of said syringe may not need a holding space for said elastic fixture, however, the rear of said blocking section has several locking tenons with reversed hooks, and said syringe body has a piston of elastic materials inside.

25. A syringe of Claim 12, wherein said elastic fixture of said syringe is composed of two interlocking pushing stoppers, a bulging block is on an extension of one stopper, said stopper is on an outer edge of said blocking section and holds onto an inner wall of said syringe body, and said two pushing stoppers adhere with each other by adhesive.

26. A syringe of Claim 12, wherein said needle socket of said syringe is installed at an outer edge of said injection needle and can be equipped with an external ring, said external ring has screw threads inside for injection needle to screw in.

27. A syringe of Claim 14, wherein said cylindrical plug of said syringe can be made of non-elastic material, said cylindrical plug has a penetration hole and uses a quick connector to replace said nozzle, there is a connection base on said transport tubing for connection of a quick connector; besides, a fastener can be set at the rear of the piston. The front of the piston plunger can have a fixation groove for the fastener. Further, inside the plunger, there can be a pressure retainer, which press is at the rear of the piston plunger.

28. A syringe of Claim 15, wherein a penetration section can replace said receptacle at the rear of said nozzle of said syringe, an outer edge of said penetration section extends outward to form a slope, said slope has several locking bulging blocks to fit tightly with an inner wall of said transport tubing when said transport tubing is inserted in said penetration section.

29. A syringe of Claim 16, wherein said balloon of said syringe can be replaced by a piston, the rear of said piston has a fixing groove, said fixing groove has several holding blocks at an opening and is used for a plunger to push in and lock in by rotation.

30. A syringe of Claim 17, wherein said concave holding space of said cap of said syringe can be replaced by a bulging holder, said bulging holder holds onto a needle connector at a front of said syringe body.

31. A syringe of Claim 17, wherein said cap of said syringe does not need to connect to said syringe body.

32. A syringe of Claim 21, wherein said piston of said syringe can be replaced by a balloon, at a front of said balloon there is a circular bulging part.

33. A syringe of Claim 22, wherein said spring coil of said syringe can be made of non-elastic materials.

34. A syringe of Claim 23, wherein said piston of said syringe can be made of non-elastic materials, there are several guiding holes in corresponding quantity and at corresponding position to a locking tenon, a wider holding groove is installed at a bottom of each guiding hole, inside said guiding groove, there is a pathway connecting to said needle socket for drug passage.

35. A syringe of Claim 23, wherein said blocking section of said syringe can be made of elastic materials, said piston has several locking tenons with reversed hooks at one side corresponding to said blocking section.

36. A syringe of Claim 25, wherein said elastic fixture of said syringe can be composed of one or more than one T stoppers and several springs, one end of said spring has a bulging block, said bulging block extends along an outer edge of said blocking section and holds onto an inner wall of said syringe body, the other end of said spring holds onto a longer side of said T stopper, at one side of said holding space, there is a groove for placing a longer extension side of said T stopper.

37. A syringe of Claim 25, wherein said elastic fixtures can be held onto said holding space of said blocking section by several springs, so two sides of said blocking section hold onto an inner wall of said syringe, said holding space has a push block at one side of each spring.

38. A syringe of Claim 25, wherein said elastic fixture can be composed of several clamping modules and a spring coil of rubber-like elastic materials, an outer edge of said spring coil holds onto an inner wall of said syringe body and an inner side of said coil spring holds onto each clamping module.

39. A syringe of Claim 27, wherein said quick connector of said syringe can be replaced by a normal connector, so said transport tubing connects to a rear of said normal connector.

40. A syringe of Claim 35, wherein said blocking section of said syringe can be made of non-elastic materials, several guiding holes are open at a corresponding position and in a corresponding quantity to said locking tenons, a wider holding groove is installed at a bottom of each guiding hole, a pathway in said holding groove can be open to connect to said needle socket for drug passage.

41. A syringe of Claim 38, wherein said clamping module of said syringe is made of a triangular elastic plate, two sides of an opening of a triangular elastic plate are bent into a spherical clamping section, inside said spherical clamping section, there is a lever that is extended to an outer edge of said triangular elastic plate, an arch elastic structure is formed at a bottom of said triangular elastic plate.
